**Europäisches Patentamt**

**European Patent Office**

**Office européen des brevets**

(11) Publication number: **0 129 228**
**A2**

## (12) EUROPEAN PATENT APPLICATION

(21) Application number: **84106882.8**

(22) Date of filing: **15.06.84**

(51) Int. Cl.³: **A 61 F 13/04**

(30) Priority: **16.06.83 US 504892**

(43) Date of publication of application:
**27.12.84 Bulletin 84/52**

(84) Designated Contracting States:
**DE FR GB NL SE**

(71) Applicant: **ISOPEDIX CORPORATION**
**17662 Armstrong Avenue**
**Irvine California 92714(US)**

(72) Inventor: **Honeycutt, Travis W.**
**8 Lago Sud**
**Irvine California 92715(US)**

(74) Representative: **Reinländer & Bernhardt Patentanwälte**
**Orthstrasse 12**
**D-8000 München 60(DE)**

(54) Improved hardenable fabric and method.

(57) A method of producing a fiber composite which is flexible but capable of being hardened without significant dimensional shrinkage and fabric produced by said fiber. As alternative embodiments, either a cross-linked and drawn mono or multi-filament fiber or a partially or fully oriented yarn is wound as a single or double helix around an elastomer, while being substantially elongated and thus under tension. Once the helix is formed, the tension imposed upon the elastomer is relaxed whereby the fabric assumes its final shape and contour. Upon the application of heat, the fabric substantially hardens without significant dimensional shrinkage.

FIG.__IA

## Description

## Improved Hardenable Fabric and Method

### Technical Field of the Invention

The present invention deals with a method of producing a fabric which is flexible but capable of being substantially hardened through the application of heat without significant dimensional shrinkage. The fabric itself is capable of being configured into divergent shapes for various uses, however, it is particularly well adapted for being configured into the shape of a tube for use as an orthosis.

### Background of the Invention

Recognizing the drawbacks inherent in the use of plaster of paris casts as well as other prior art cast materials, a fabric is disclosed which is flexible but capable of being substantially hardened through the application of heat. The fabric uniquely adapted as a substitute for prior cast materials by exhibiting rigidity but not the secondary characteristics of prior cast materials, which have resulted in patient discomfort and even physical disorders.

Although the disclosed product is far superior than any hardenable fabric previously known, it has been determined that it could be improved by minimizing fabric shrinkage and increasing fabric stiffness upon the application of heat by increasing the density of hardenable fiber in the fabric.

## Summary of the Invention

The present invention deals with a fabric and method of producing the fabric which is flexible but capable of being substantially hardened through the application of heat without significant dimensional shrinkage. The method includes providing a second fiber which, prior to being incorporated with the first fiber, is impressed with a memory so that upon the application of heat, the fiber is inclined to become shorter in length, thicker and significantly harder and less pliable.

Once the second fiber has been preconditioned to exhibit these properties, it is helically wound about a first fiber. Although virtually any means is contemplated for forming the helix, it is basic to the present invention that the first fiber, which is preferably an elastomer, is helically wound with the second fiber while being substantially elongated and thus under tension. Once the helix is formed, the tension imposed upon the elastomer is substantially lessened or removed.

Once the above has been accomplished, the composite fibers or yarns are ready to be knitted or woven to produce the hardenable fabric. It is contemplated that this can be done in one of two ways. As a first embodiment, the composite fibers can simply be woven or knitted by conventional techniques well known to those skilled in the textile fabrication art, such as by circular or warp knitting. More preferably, however, the composite fibers can be combined with yet

0129228

a second elastomer which is combined with the helically formed composite fibers, while the second elastomer is under tension. Once the knitting or weaving operation is completed, the tension imposed upon the second elastomer is substantially lessened or removed, resulting in a tilt-stitch pattern.

The achievement of the results can be more fully appreciated when considering the following description and appended drawings wherein:

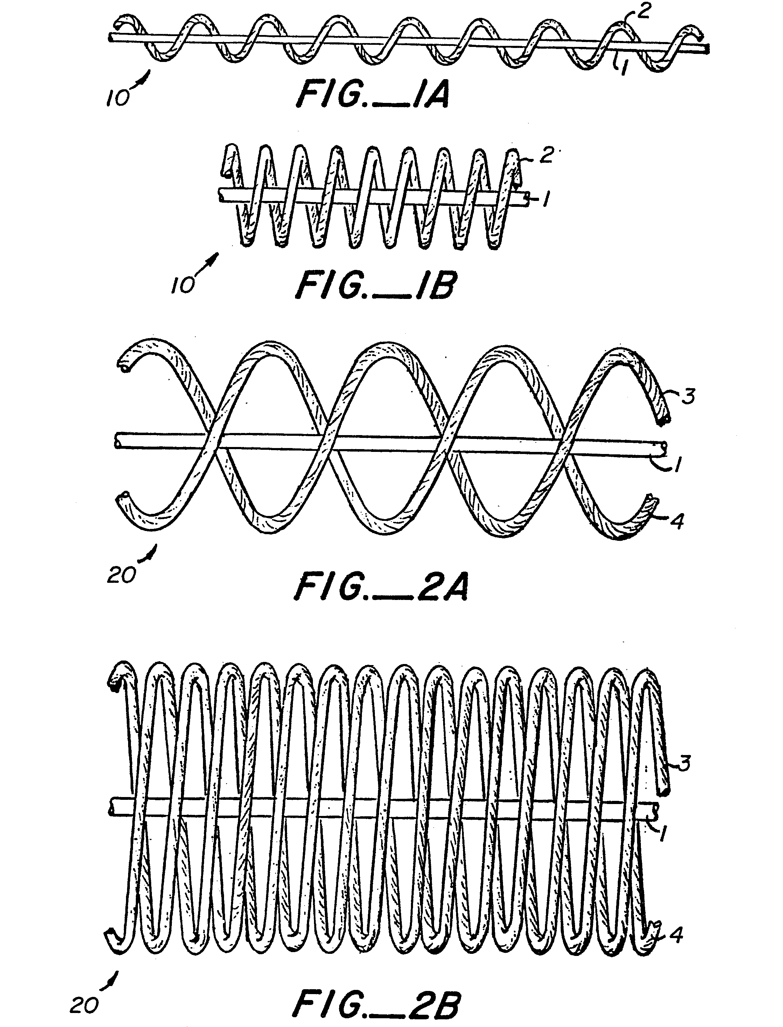

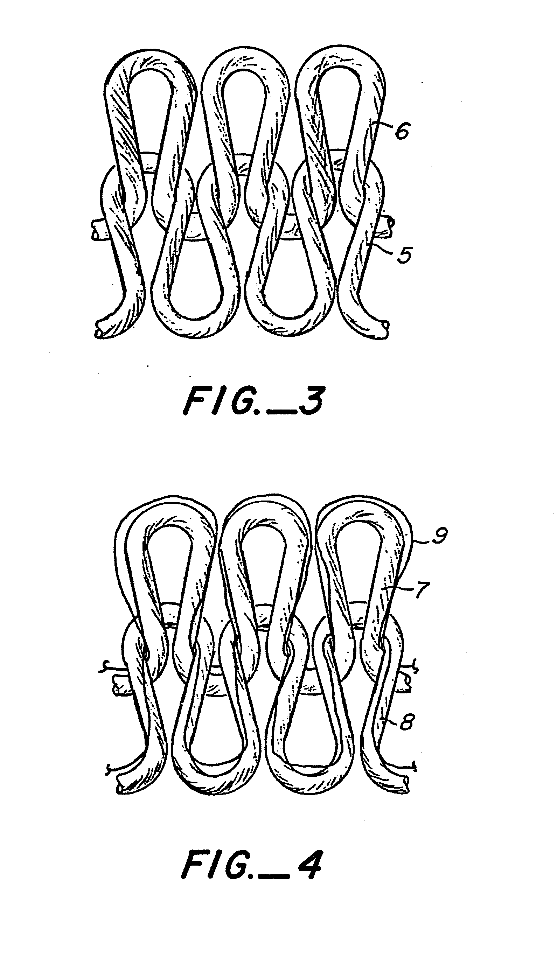

Figs. 1A, 1B, 2A and 2B depict plan views of two embodiments of the composite yarn or fiber of the present invention and Figs. 3 and 4 depict two embodiments of fabric made from the composite fibers disclosed herein.

Detailed Description of the Invention

Turning to Fig. 1A, elastomer 1 is shown being combined with fiber 2 to form composite fiber 10. As noted above, elastomer 1 is wound or wrapped by helix 2 while under tension, which is relaxed after the helix is formed. This results in compaction of fiber 2 resulting in the configuration depicted in Fig. 1B.

The method of forming the helix of fabric 2 about elastomer 1 is well known to those skilled in the art. Broadly, the helix can be formed by either a wrapping technique or a twisting technique employing conventionally available devices, such as Sauer-Allma Novelty Yarn Equipment type ESP-F. By using such devices, the helix can be formed while elastomer 1 is under tension (Fig. 1A), which is substantially lessened or eliminated after helix formation (Fig. 1B).

It is essential to the present invention that fiber 2 be impressed with a memory by either of two general methods. As a first alternative, fiber 2 is composed of a single or multi-filament material capable of cross-linking. The fiber is exposed to radiation to so cross-link a substantial fraction of the molecules of the fiber, whereupon the fiber strands are drawn to substantially increase their length and cause unrelieved strain to be formed in the molecular infrastructure. In employing this method, it is contemplated that fiber 2 be a member selected from the group consisting of a polyolefin, such as a polyethylene or polypropylene, a polyamide, a polyester, a polyacrylonitrile, a polyaramid, a cellulosic and a polyimidazole. A cross-linking agent can be incorporated to enhance the degree and extent of cross-linking, composed of, for example, substituted vinyls and acetylides.

As an alternative, fiber 2 can be produced by merely drawing a mono or multi-filament yarn at a temperature above room temperature to substantially cause at least partial orientation of the polymeric crystals of the fiber. Tension is maintained in the fiber until it has had an opportunity to set or cure, and thus the elongation and induced unrelieved strain is retained. This second embodiment contemplates a fiber selected from the group consisting of a polyolefin, such as polyethylene and polypropylene, a polyacrylonitrile, a polyamide, a polyaramide and, preferably, a polyester. Broadly, the first fiber is drawn approximately 1.1 to 8 times its undrawn length, more preferably 1.5 to 6 times its undrawn length, and ideally 1.75 to 3 times its undrawn length.

As a second embodiment of the present invention, a double helix of hardenable fibers 3 and 4 can be wrapped or twisted about elastomer 1 as shown in Fig. 2A, while said elastomer is under tension and thus substantially elongated. Upon the formation of composite 20, the tension placed upon elastomer 1 is substantially lessened or removed resulting in compaction of the composite as shown in Fig. 2B.

Fibers 3 and 4 can be of the same or different chemical composition, each selected from the groups recited above.

The composite fibers, once formed, either of a single helix configuration (Fig. 1B) or of the double helix confirmation (Fig. 2B) can be knitted or woven by techniques well known to those skilled in the textile fabrication art. For example, both circular and warped knitting are contemplated for use in practicing the present invention.

As a first embodiment, composites 10 or 20 can be woven or knitted into the basic stitching pattern as shown in Fig. 3. More specifically, composites 5 and 6, which are, in effect, either composites 10 or 20 of Figs. 1B or 2B are shown knitted or woven into multiple rows forming a fabric pursuant to the present invention.

As an alternative, the fabric as shown in Fig. 4 can be constructed by knitting or weaving the composite fibers with elastomer 9, which, again, is elongated and under tension during the weaving or knitting operation, said tension being relaxed or removed after the weaving or knitting operation is completed. The fabric depicted in Fig. 4·results in a unique construction characterized as a "tilted stitching pattern", which is particularly adaptable for use as an orthosis.

The combination of composite fibers 7 and 8 with elastomer 9 into a woven pattern as illustrated in Fig. 4 results in the unique interaction resulting from the properties of each fiber. Composites 7 and 8 display the physical characteristics of maintaining their relative pliability and physical dimensions until subjected by sufficient heat to loosen the molecular structure of the helically wound fiber 2, 3 and 4 of Figs. 1B and 2B, which causes the drawn fiber to contract longitudinally and at the same time, substantially harden. But the application of heat does not materially affect either elastomers 1 or 9. Because both elastomers were woven or knitted under tension, which was removed on the one hand after forming the fiber and on the other after the fabric was prepared, the shrinking and hardening of the fabric does not substantially reduce the fabric's physical dimension due to its compaction by the elastomer. These characteristics are enhanced due to the helical nature of the composites forming thicker more elastic hardenable fabric, resulting in superior fabric body with less dimensional change upon fabric hardening.

The present invention teaches also forming single or double helixes of hardenable yarn about the elastomer. The purpose of this is to increase the length or amount of hardenable yarn in a given stitch length in order to reduce fabric shrinkage and increase fabric stiffness when the fabric is exposed to heat. The helix can be formed by employing a conventional double hollow spindle yarn wrapping machine whereby the elastomer is loaded onto the creel holder and threaded through the hollow spindle to the final yarn wind-up rolls. The elastomer can be 40 to 1200 denier, more preferably 70 to 560 denier or most advantageously 140 to 360 denier mono or multi-filament spandex or rubber yarn, such as Lycra Spandex available from E. I. Du Pont de Nemours & Co. Although the core need not be limited to an elastomer, the filament must possess the property of extending substantially under tension while the helix is formed and exhibiting hysteresis by contracting once tension is removed thus providing a compaction of the composite fiber.

Continuing the manufacturing procedure, it is noted that the afore-mentioned hollow spindles are pre-loaded with the hardenable yarn using conventional winding techniques. The hardenable yarn, comprised of materials as recited above, preferably should be 40 to 1,000 denier, more preferably 70 to 700 denier and most advantageously 150 to 500 denier mono or multi-filament yarn. As illustrative of commercially available products ideally adapted for use in practicing the present invention are 270/30 denier/filaments and 490/30 denier/filaments polyester POY yarns available from Eastman Kodak Co.

The machine gearing is set up such that the hardenable yarn is caused to be wrapped around the stretched elastomer as it is pulled through the machine. When forming the double helix, the hollow spindles are rotated in opposite directions to each other while the elastomer is stretched 1.5 to 10 times, more preferably 1.75 to 6 times, and most preferably 2 to 4 times its at rest length as it travels through the hollow spindles when constrained by the bottom and top nips of the feed rolls which are turning at different rotational speeds. Naturally, for formation of single helix yarns, only one hollow spindle is loaded with hardenable yarn. The rotational speed of the hollow spindles is such that 2 to 12, and more preferably 4 to 10, and most desirably 6 to 8 turns of hardenable yarn per linear length of elastomer results when said elastomer is untensioned and at rest (Figs. 1B and 2B).

When the fabric of the present invention is configured into a tubular shape, an ideal orthosis is formed. When configured over an appendage, such as an arm or a leg, which is in need of such an orthosis, and subjected to heat through the use of, for example, a heat gun, the fabric is caused to substantially harden without significant dimensional shrinkage. This provides an excellent substitute for a plaster of paris cast having the sufficient and necessary hardness and strength characteristics necessary for such an orthosis, yet without displaying any of the drawbacks inherent in the use of plaster of paris.

## Claims

1.  A method of producing a fabric which is flexible but capable of being substantially hardened without significant dimensional shrinkage comprising:

A. providing a first fiber capable of shrinking and substantially hardening upon the exposure of said first fiber to heat;

B. forming a helix of said first fiber around a second fiber, said second fiber being an elastomer which is combined with said first fiber while being substantially elongated and thus under tension;

C. completing the helix-forming operation of step B at which time the tension imposed upon the elastomer is substantially lessened or removed; and

D. knitting or weaving a composite comprising said first and second fibers into a final fabric.

2.  The method of claim 1 wherein a double helix of said first fiber is formed around said second fiber, while said second fiber is substantially elongated and thus under tension.

3. A method of producing a fabric which is flexible but capable of being substantially hardened without significant dimensional shrinkage comprising:

A. providing a first fiber capable of shrinking and substantially hardening upon the exposure of said first fiber to heat;

B. forming a helix of said first fiber around a second fiber, said second fiber being an elastomer which is combined with said first fiber while being substantially elongated and thus under tension;

C. completing the helix-forming operation of step B at which time the tension imposed upon the elastomer is substantially lessened or removed;

D. knitting or weaving the composite comprising said first and second fibers with a third fiber, said third fiber being another elastomer which is knitted or woven with said composite while being substantially elongated and thus under tension; and

E. completing the knitting or weaving operation of step D at which time the tension imposed upon the third fiber is substantially lessened or removed.

4. The method of claim 3 wherein a double helix of said first fiber is formed around said second fiber while said second fiber is substantially elongated and thus under tension.

5. The method of claims 1 or 3 wherein the fabric is configured in the shape of a tube.

6.    The method of claim 5 wherein the tube is configured for use as an orthosis.

7.    The method of claims 1 or 3 wherein said second fiber is stretched approximately 1.5 to 10 times its unstretched length while forming said helix of first fiber.

8.    The method of claims 1 or 3 wherein said second fiber is stretched approximately 1.75 to 6 times its unstretched length while forming said helix of first fiber.

9.    The method of claims 1 or 3 wherein said second fiber is stretched approximately 2 to 4 times its unstretched length while forming said helix of first fiber.

10.    The method of claims 1 or 3 wherein said first fiber is wrapped as a helix around said second fiber to achieve approximately 2 to 12 turns of said first fiber per inch of said second fiber as measured when the tension on said second fiber has been substantially lessened or removed.

11.   The method of claims 1 or 3 wherein said first fiber is wrapped as a helix around said second fiber to achieve approximately 4 to 10 turns of said first fiber per inch of said second fiber as measured when the tension on said second fiber has been substantially lessened or removed.

12.   The method of claims 1 or 3 wherein said first fiber·is wrapped as a helix around said second fiber to achieve approximately 6 to 8 turns of said first fiber per inch of said second fiber as measured when the tension on said second fiber has been substantially lessened or removed.

13.   The fabric produced by the methods of claims 1, 2, 3 or 4.

14.   A method of forming a cast from the fabric of claim 13 comprising slipping a tube of said fabric over an extremity requiring the cast and applying sufficient heat to the fabric to substantially harden the same.

15.   The method of claim 14 wherein the heat is applied non-uniformly to selectively harden certain areas of the fabric more than others.

FIG.__IA

FIG.__IB

FIG.__2A

FIG.__2B

*FIG.__3*

*FIG.__4*